# EUROPÄISCHE PATENTSCHRIFT

(11) **EP 1 786 481 B1**
(45) Veröffentlichungstag und Bekanntmachung des Hinweises auf die Patenterteilung: **11.11.2009**
(21) Anmeldenummer: 05778361.5
(22) Anmeldetag: 01.09.2005
(51) Int. Cl.: A61L 15/40, A61L 15/44, A61L 26/00, A61F 13/00, A61N 1/16, A61N 5/00

(54) **KOMPRESSE FÜR DIE KOMBINIERTE APPLIKATION VON NATURHEILSTOFFEN UND ELEKTROMAGNETISCHEN STRAHLUNGEN**
COMPRESS FOR THE COMBINED APPLICATION OF NATURAL CURATIVES AND ELECTROMAGNETIC RAYS
COMPRESSE POUR L'APPLICATION COMBINEE DE MATIERES CURATIVES NATURELLES ET DE RAYONS ELECTROMAGNETIQUES

(30) Priorität: 06.09.2004 DE 102004043451
(43) Veröffentlichungstag der Anmeldung: 23.05.2007
(73) Patentinhaber: Vollert, Kai, 8280 Kreuzlingen (CH)
(72) Erfinder: VOLLERT, Hegall, 78315 Radolfzell (DE)
(86) Internationale Anmeldenummer: PCT/IB2005/002594
(87) Internationale Veröffentlichungsnummer: WO 2006/027657

(56) Entgegenhaltungen:
- EP-A- 1 074 275
- WO-A-2004/039358
- CN-A- 1 085 770
- CN-A- 1 101 578
- US-A1- 2002 156 340
- US-A1- 2003 068 614
- US-A1- 2003 069 618
- US-B1- 6 312 450

## Beschreibung

### Gebiet der Erfindung

Die Erfindung betrifft eine Kompresse zur kombinierten Applikation mindestens zwei verschiedener Naturheilverfahren durch Anbringung am Körper eines Patienten.

Kompressen im Sinne der Erfindung sind Grundkörper in Form von Umschlägen, Auflagen, Manschetten, Bandagen oder dergleichen für bestimmte Körperteile zur therapeutischen Behandlung von äußerlichen und innerlichen Beschwerden.

Ein Extrakt im Sinne der Erfindung ist ein aus einem natürlichen Material (Mineral, Pflanze, Tier) herausgelöster Stoff.

Unter Essenz wird im Folgenden eine konzentrierte Lösung eines aus Mineralien, Pflanzen oder Tieren gewonnenen Extrakts verstanden. Eine Essenz kann flüssig, pastös oder rieselförmig sein.

Mit Naturheilstoff werden im Folgenden Heilkräuter, Heilpflanzen, Essenzen, Extrakte, homöopathische Arzneimittel, Bachblüten und andere biologische Wirkstoffe bezeichnet.

### Stand der Technik

Kompressen sind ein altbekanntes Heilmittel zur Behandlung verschiedener Krankheiten und Beschwerden, wie beispielsweise rheumatische Beschwerden, Muskelverspannungen, Muskelkater, zur Senkung von Fieber etc.

Seit einiger Zeit gewinnen Methoden der Naturheilkunde als Alternative bzw. als Ergänzung zur Schulmedizin zunehmend an Bedeutung. So wird heute von vielen Menschen zur Selbstbehandlung und in zahlreichen medizinischen oder psychologisch orientierten Praxen und Institutionen eine Form der Naturheilkunde, die sog. klassische Homöopathie nach Dr. Samuel Hahnemann, angewandt. Diese setzt Arzneien vorwiegend aus dem Mineral-, Pflanzen- und Tierreich ein, welche potenzierte, das heißt, verdünnte und verschüttelte (dynamisierte, energetisch verstärkte) Wirkstoffe enthalten. Der Begriff "Homöopathie" stammt aus der griechischen Sprache und kann mit "ähnliches Leiden" übersetzt werden. Damit ist gemeint, dass in der Homöopathie eine Erkrankung mit einer kleinen Menge eines Naturstoffs behandelt wird, die am gesunden Menschen in größerer Dosis verabreicht, zu ähnlichen Symptomen führt, wie sie für eine bestimmte Erkrankung charakteristisch sind. Für die Herstellung von homöopathischen Arzneimitteln werden die pflanzlichen, tierischen oder mineralischen Extrakte zuerst in eine flüssige (Urtinktur) oder in eine pulverisierte Form (Verreibung) gebracht. Anschließend werden diese mit einer Trägersubstanz (flüssige meist mit einer Alkohol-Wasser-Mischung, feste mit Milchzucker) vermischt. Dabei werden Wirksubstanzen und Trägerstoffe in bestimmten Verdünnungsverhältnissen geschüttelt oder verrieben. Dieser Vorgang wird so lange fortgesetzt, bis die gewünschte Potenz des Heilmittels erreicht ist. Durch die Potenzierung nimmt die materielle Substanz ab, die für die Heilwirkung verantwortlichen Kräfte werden jedoch gesteigert.

Homöopathische Arzneimittel werden in der Regel über die Haut oder oral appliziert. Es ist darüber hinaus in der Homöopathie anerkannt, dass Arzneimittel mit einer hohen Potenz auch ohne direkten Kontakt mit dem menschlichen Körper ihre heilende Wirkung entfalten können.

Die nach dem englischen Arzt und Forscher Dr. Edward Bach als "Bach-Blüten-Therapie" bezeichnete Form der klassischen Homöopathie behandelt körperliche Krankheiten mittels Arzneien, die aus Extrakten der Blüten bestimmter Pflanzen hergestellt werden. Bach-Blüten werden regelmäßig aufgelöst in Tinkturen, Tropfen (flüssig) oder als Salbe (pastös) verabreicht. Genauso ist die Verabreichung getrockneter Bach-Blüten und rieselförmiger Blütenextrakte bekannt.

Eine weitere Form der alternativen Naturheilkunde ist die Farbtherapie. Diese basiert auf der Erkenntnis, dass jede Spektralfarbe eine typische Wellenlänge und Energie besitzt, die auf den Körper übertragen werden kann. Rotes Licht zum Beispiel wirkt wärmend und anregend, blaues Licht kühlend und beruhigend. Bei der Farbtherapie versucht man, die Kräfte der Farben vorbeugend oder heilend einzusetzen. Dabei ist es anerkannt, dass die Farben nicht nur über die visuelle Wahrnehmung ihre Wirkung entfalten, sondern auch durch direkten Kontakt des menschlichen Körpers mit farbigem Material heilende Wirkungen bei Beschwerden und Erkrankungen wie beispielsweise rheumatischen Beschwerden, Muskelverspannungen, Kopfschmerzen, depressiven Verstimmungen, Schlaflosigkeit, Nervosität, Angst, Schwächezustände oder Konzentrationsschwierigkeiten entstehen können.

Die sog. "Neue Homöopathie" nach Erich Körbler schließlich baut auf der Erkenntnis auf, dass jedes Element im Universum auf einer bestimmten Wellenlänge eine Strahlung (Information) aussendet. Ein lebender Organismus ist daher gleichzeitig vielen Strahlungen unterschiedlicher Intensität und Qualität (Nachrichteninhalt) ausgesetzt. Beim Menschen angekommen, üben die Informationen einen speziellen Reiz aus, der wiederum zu einer Reizreaktion führt. Mittels energetischer Ladungsmuster, die in Form bestimmter geometrischer Formen an oder in der Nähe des menschlichen Körpers angebracht werden, versucht die Neue Homöopathie, die unverträglichen Informationen zu löschen und positiv wirkende (heilende) energetische Informationen zu übertragen. Bei den energetischen Informationen wird die reine Energie benutzt, nicht eine Energie aussendende Materie.

Herkömmliche Kompressen entfalten ihre heilende Wirkung durch Einsatz von Wärme bzw. Kälte oder durch Stabilisierung der erkrankten oder verletzten Körperteile oder Gelenke. Aus dem Stand der Technik sind überdies verschiedene Kompressen bekannt, die eine gleichzeitige Applikation physikalischer und medikamentöser Therapien ermöglichen.

So ist aus DE 31 18232 A1 ein Hautverband mit einer auf der Haut aufliegenden Grundschicht und einer Befestigungsvorrichtung bekannt, die eine Deckschicht aufweist, welch die Grundschicht derart überspannt, dass zwischen Grund- und Deckschicht eine Kammer gebildet wird.

EP 0 360 270 A2 offenbart eine Kompresse für die Kombination eines Kompressionsverbandes mit der Anwendung von Kälte, die aus drei Hauptkomponenten, nämlich einer Bandage, einem Kühlmittelkissen und einem jeweils nach Bedarf ausgewählten Wirkstoffkissen besteht.

DE 199 92 369 A1 beschreibt eine Vorrichtung für die Anwendung in der Bion- oder Orgontherapie, die durch Vermischung von Quarzsand, der zuvor mit natürlichem oder künstlichem Licht bestrahlt wurde, und aufgeschmolzenem Kunststoffgranulat hergestellt und anschließend in die Form einer Kompresse, Auflage, Bandage oder Ähnliches gebracht wird.

Die Druckschrift CN 1 085 770 zeigt eine Heilungsvorrichtung, die im Wesentlichen ein bewegliches Kissen zur Aufnahme von chinesischen Kräutern umfasst. Zusätzlich sind Vorrichtungen vorgesehen, die ein Aufheizen, ein Kühlen sowie die Abgabe von elektromagnetischer Strahlung durch das Kissen ermöglichen. Das Kissen selbst ist volumenmässig bestimmt und weist die Form eines Kissens auf, wobei metallische Platten vorgesehen sind, um die elektromagnetische Strahlung auf die chinesischen Kräuter zu übertragen. Die Kräuter sind lose in dem Kissen bevorratet.

Aus der CN 1 101 578 ist ebenfalls ein therapeutisches Kissen bekannt, dass zur Aufnahme von einer Vielzahl von chinesischen Kräutern dient. Eine elektrische Schaltung sowie Einstelleinrichtung und Kontaktelemente, die die elektrische Verbindung zu den Kräutern herstellen, dient dazu, insbesondere heilende Eigenschaften der chinesischen Kräuter auszulösen und diese transdermal zu applizieren. Das Kissen weist ein entsprechendes Volumen auf und umfasst metallische Elektroden, die in das Kissen hineinreichen. Die Kräuter sind lose in dem Kissen bevorratet.

Aus der US 6,312,450 ist ein System und ein Verfahren zur Verbesserung der Hauteigenschaften beschrieben. Hierin wird eine kosmetische Vorrichtung vorgeschlagen, die es ermöglicht, Licht, beispielsweise Laserlicht, mit bestimmten Wellenlängen auf die Haut zu applizieren. Hierfür wird eine Kompresse bereitgestellt, die Licht in Ausbildung der elektromagnetischen Wellen auf die Hautoberfläche überträgt, jedoch auch gewisse Wellenlängen absorbiert, so dass sie keine Schädigung auf der Haut ausüben.

Aus der EP 1 074 275 ist eine Vorrichtung bekannt, mittels der elektromagnetische Strahlung gezielt auf die Haut übertragen werden kann. Hierzu werden Einrichtungen vorgeschlagen, die Lichtemittenten auf deren Umfang bereitstellen, damit das Licht entsprechend auf die Haut emittieren kann.

In der US 2002/0156340 ist eine Vorrichtung vorgeschlagen, mittels der entsprechende Nährstoffe transdermal appliziert werden können.

Aus der US 2003/0068614 ist eine Vorrichtung bekannt, die aus mehreren Elementen besteht, wobei ein Element ein entsprechendes Reservoir aufweist, um zu applizierende Stoffe aufzunehmen. Nicht flexible Platten, die vorzugsweise magnetisch sind, dienen dazu, aus den drei Komponenten eine geschlossene Vorrichtung bereitzustellen, die zur transdermalen Verabreichung des Inhaltes des Reservoirs geeignet ist.

Aus dem Stand der Technik sind bislang keine Vorrichtungen bekannt, die es erlauben, die zuvor beschriebenen Therapieformen der Naturheilkunde, nämlich Farbtherapie, Bach-Blüten-Therapie, klassische homöopathische Heilmethoden nach Dr. Samuel Hahnemann sowie neue homöopathische Heilmethoden nach Erich Körbler kombiniert am menschlichen Körper anzuwenden.

### Nachteile des Standes der Technik

Bislang werden die in der Neuen Homöopathie nach Erich Körbler verwendeten energetischen Ladungsmuster derart eingesetzt, dass diese Codes auf laminiertes Papier aufgetragen und dann in die unmittelbare Nähe des Therapiepatienten verbracht werden. Nachteil dieses laminierten Papiers ist es, dass es nicht unmittelbar am Körper, auf der Haut getragen werden kann, da es bei Bewegungen des Patienten zerknüllt wird.

Ähnliche Nachteile treten bei der Verwendung von Webstoffen auf, da hier die Strichcodes am Körper verrutschen oder durch Faltenwurf verschoben werden können.

Herkömmliche Kompressen für die Wärme-/Kältebehandlung sind bislang nur für die Aufnahme von flüssigen oder pastösen Stoffen vorgesehen. Sollen diese Kompressen mit rieselförmigen Naturheilstoffen, beispielsweise Bach-Blüten-Essenzen gefüllt werden, besteht ein wesentlicher Nachteil darin, dass aufgrund der geringen Dichte der rieselförmigen Naturheilstoffe die Füllungen der Kompressen je nach Position derselben im Raum verrutschen und ungleichmässig auf der zum Körper des Patienten zugerichteten Seite aufliegen.

Zudem kann es geschehen, dass die vorbekannten Kompressen aufgrund der Körnigkeit der rieselförmigen Naturheilstoffe oder einer schlechten Schweissnaht der Kunststoffkompressen reißen und die Essenzen dadurch unkontrolliert austreten können. Dies kann insbesondere dann geschehen, wenn die Kompressen durch eine mechanische Belastung, wie etwa durch Körperbewegung hervorgerufen, beschädigt werden.

### Aufgabe

Die Aufgabe der Erfindung besteht daher darin, eine Vorrichtung zu schaffen, die es ermöglicht, mindestens zwei der oben genannten naturheilkundlichen Therapieformen gemeinsam am Körper des Menschen anzuwenden.

### Lösung der Aufgabe

Zur Lösung der Aufgabe wird vorgeschlagen, dass die rieselförmigen Naturheilstoffe, beispielsweise Bach-Blüten, mit Kautschuk, Silikon, Kunststoff oder einem ähnlichen Material vermischt werden und diese Mischung in Form einer Kompresse, Bandage, Auflage oder eines Pflasters gegossen, gepresst oder gezogen wird.

Wahlweise kann eine solche Kompresse mit einer Farbe entsprechend der Farbtherapie und/oder einem energetischen Ladungsmuster gemäß der neuen Homöopathie nach Erich Körbler versehen werden.

Wahlweise kann zusätzlich oder anstatt der getrockneten Bach-Blüten-Essenzen ein oder mehrere homöopathische Naturheilstoffe mit dem Kautschuk, Silikon, Gummi oder anderem vergleichbaren Material vermischt werden.

### Vorteile der Erfindung

Ein wesentlicher Vorteil der Erfindung besteht darin, dass die erfindungsgemäße Vorrichtung am menschlichen Körper getragen werden kann und trotz Bewegung des Körpers eine im Vergleich zu anderen Materialien wie Stoff oder Papier, stabile Oberfläche beibehält, die zur Aufnahme eines energetischen Ladungsmusters nach der Neuen Homöopathie von Erich Körbler und/oder als Medium für die Farbtherapie dient.

Ein weiterer Vorteil ist, dass die rieselförmigen Naturheilstoffe derart aufbewahrt werden können, dass die Vorrichtung durch mechanische Belastungen nicht beschädigt werden können, was zu einem Austreten der Essenzen führen könnte.

Ein weiterer Vorteil besteht darin, dass die rieselförmigen Naturheilstoffe durch den sie umschließenden Kautschuk, Silikon, Kunststoff oder ein ähnliches Material fest umschlossen sind und somit auch bei mechanischen Bewegungen und damit verbundenen Verformungen der Kompresse nicht ihre relative Position innerhalb der Kompresse verlieren, d.h. nicht verrutschen können.

Eine alternative Ausführung der Vorrichtung sieht einen thermoplastischen oder wärmeschrumpfenden Kunststoff vor. Dadurch ist es möglich, dass in einem ersten Schritt die Vorrichtung an ein entsprechendes Körperteil angepasst wird und durch Wärmebehandlung, beispielsweise mit einem Föhn, derart verändert wird, dass die Vorrichtung ihre so erhaltene Form behält.

Die Verwendung formbarer Materialien erlaubt es überdies, dass unmittelbar beim Herstellungsprozess an die Seiten der Vorrichtung Verschluss- oder Halteelemente einteilig ausgebildet werden können. Diese Verschluss- oder Halteelemente können derart ausgestaltet sein, dass Haltevorrichtungen, beispielsweise gummiartige Bänder, an die Verschluss- oder Halteelemente angebracht werden können, um die gesamte Vorrichtung an einem Körperteil zu befestigen.

Eine alternative Lösung sieht vor, dass die Vorrichtung in zwei Verfahrensschritten hergestellt wird, wobei zunächst in eine dünne Schicht erhitzten Kautschuks, Silikons, Kunststoffs oder in ein ähnliches Material die rieselförmigen Naturheilstoffe eingestreut werden und in einem weiteren Schritt die nach dem ersten Verfahrensschritt noch nicht vollständig umschlossenen rieselförmigen Naturheilstoffe mit einer zweiten halbdurchlässigen Kunststoffschicht oder einem ähnlichen Material überzogen werden, wodurch ein Austreten der Wirkstoffe der Naturheilstoffe durch die zweite Kunststoffschicht in Richtung der Haut des Patienten erfolgen kann, ohne dass Feuchtigkeit, beispielsweise menschlicher Schweiß, in die Kompresse eindringen kann.

Da die rieselförmigen Naturheilstoffe nicht in Kautschuk, Silikon, Latex oder einem ähnlichen Kunststoff aufgelöst werden, besteht ein weiterer Vorteil darin, dass mit der Vermischung der rieselförmigen Naturheilstoffe mit Kautschuk, Silikon, Kunststoff oder ähnlichen Materialien keine Verdünnung der Essenzen, und damit keine Verringerung der Heilwirkung verbunden ist.

Ein weiterer Vorteil der Erfindung besteht darin, dass die verwendeten Materialien Kautschuk, Silikon oder Kunststoff sterilisiert, gekocht oder in einer Seifenlauge, beispielsweise in einer Waschmaschine, gewaschen werden können und dadurch wiederholt eine hygienische Anwendung der Vorrichtung auf der menschlichen Haut erlauben.

Eine weitere alternative Lösung sieht vor, dass die Kompresse aus Kautschuk, Silikon, Kunststoff oder einem ähnlichen Material in Form einer Tasche ausgebildet wird, in welche eine Einlage eingebracht werden kann, die aus einem hydroskopischen Material, beispielsweise Baumwolle besteht und dieses Material zuvor in eine flüssige Essenz eines Naturheilstoffes getränkt wird. Alternativ kann die Einlage auch durch den Kautschuk, das Silikon, den Kunststoff oder ein ähnliches Material vollständig umschlossen werden.

Schließlich wird in einer weiteren alternativen Ausführung eine erste Schicht der Kompresse aus Kautschuk, Silikon, Kunststoff oder einem ähnlichen Material derart ausgebildet, dass auf der gesamten Fläche der Kompresse Kammern definiert werden, die durch Stege voneinander getrennt sind. In die derart definierten Kammern können die Naturheilstoffe in flüssiger, pastöser oder rieselförmiger Form eingefüllt und in einem weiteren Verfahrensschritt die derart befüllte Kompresse mit einer Kautschuk-, Silikon- oder Kunststoffschicht oder mit einem ähnlichen Material verschlossen werden. Vorzugsweise besteht die zweite, abschließende Schicht aus einer halbdurchlässigen Membran, die ein Austreten der Naturheilstoffe an der dem menschlichen Körper zugewandten Seite der Kompresse erlaubt, die Aufnahme von Flüssigkeit in die Kompresse jedoch verhindert.

### Kurze Beschreibung der Zeichnungen

Es zeigen:
- Fig. 1: Eine schematische Darstellung einer erfindungsgemäßen Kompresse,
- Fig. 2: eine schematische Darstellung einer alternativen Ausführungsform der Kompresse gemäß Fig. 1.

### Ausführliche Beschreibung eines Ausführungsbeispiels

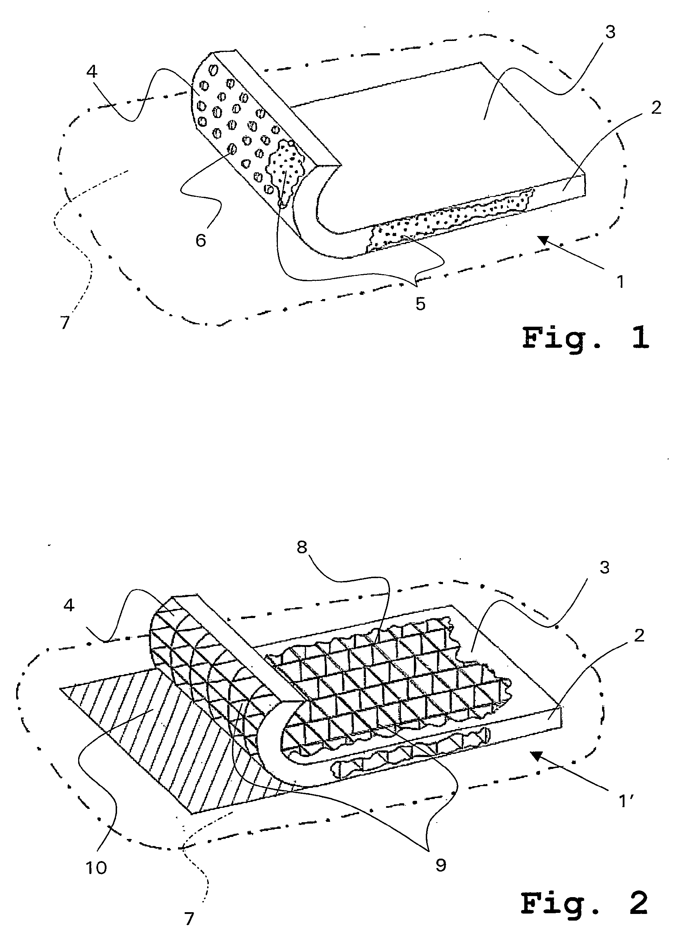

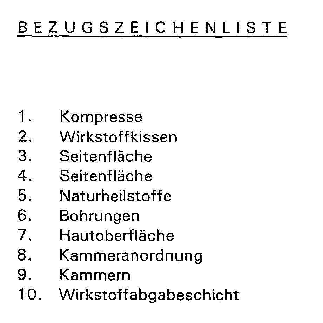

Figur 1 zeigt die erfindungsgemäße Kompresse 1 bestehend aus dem Wirkstoffkörper 2, mit einer der Hautoberfläche 7 eines Benutzers (nicht dargestellt) abgewandten Seite 3 und einer der Hautoberfläche 7 zugewandten Seite 4. Der Wirkstoffkörper 2 besteht erfindungsgemäß aus Silikon, Kautschuk, Gummi oder einem vergleichbaren Kunststoff, in welchen in dem dargestellten Ausführungsbeispiel die transdermal zu verabreichenden Naturheilstoffe 5 eingemischt worden sind. Zur Herstellung dieses Wirkstoffkörpers kann ein Verfahren mit folgenden Verfahrensschritten angewendet werden:
Vermischen der Naturheilstoffe 5 mit Kunststoffgranulat, Kautschukgranulat, Silikongranulat oder einem ähnlichen Material, Aufschmelzen des Granulats, Einfüllung des aufgeschmolzenen Granulats zusammen mit den darin enthaltenen Naturheilstoffen 5 in die gewünschte Form des Wirkstoffkissens 2.

Alternativ können die Naturheilstoffe 5 auch in die erhitzte Granulatmasse eingestreut oder eingerührt und anschließend in der gewünschten Form zur Erkaltung gebracht werden.

Um eine fluidmässige Verbindung zwischen den Naturheilstoffen 5 und der Hautoberfläche 7 herzustellen, weist die Kompresse vorzugsweise an der der Hautoberfläche 7 zugewandten Seitenfläche 4 Öffnungen 6 auf.

Erfindungsgemäß wird mindestens eine der Seitenflächen 3 oder 4 mit einem energetischen Ladungsmuster nach der neuen Homöopathie nach Erich Körbler versehen oder aus einem Material mit einem Lichtemissionsspektrum von 390 bis 740 nm ausgebildet. Derart können die therapeutischen Wirkungen der Neuen Homöopathie nach Erich Körpbler oder der Farbtherapie zeitgleich mit den eingebrachten Naturheilstoffen 5 am Körper des Patienten appliziert werden. Alternativ können sowohl energetische Ladungsmuster, als auch ein Material mit einem zuvor genannten Lichtemissionsspektrum zusammen verwendet werden.

Figur 2 zeigt eine Kompresse 1' als alternative Lösung der Aufgabe, die im Wesentlichen aus einem Wirkstoffkissen 2, mit einer der Hautoberfläche 7 eines Benutzers (nicht dargestellt) abgewandten Seite 3 und einer der Hautoberfläche 7 zugewandten Seite 4. Das Wirkstoffkissen wird dabei erfindungsgemäß von einer Kammeranordnung 8 durchzogen, die aus einer Vielzahl voneinander abgeschlossener Kammern 9 besteht. In diese Kammern 9 werden die Naturheilstoffe 5 (in Fig. 2 nicht dargestellt) eingebracht. Das Wirkstoffkissen 2 ist dabei zu der der Hautoberfläche 7 zugewandten Seitenfläche 4 geöffnet. Um ein Austreten der Naturheilstoffe 5 zu verhindern, wird die Seitenfläche 4 durch ein Wirkstoffabgabeschicht 10 verschlossen, welche die Eigenschaft aufweist, dass die Naturheilstoffe 5 durch die Wirkstoffabgabeschicht 10 diffundieren und derart in Kontakt mit der Hautoberfläche 7 treten können. Der Diffusionskoeffizient kann dabei durch die Materialauswahl der Wirkstoffabgabeschicht 10 und/oder des Naturheilstoffes 5 beeinflusst werden. Erfindungsgemäß weist mindestens eine der Seitenflächen 3 oder 4 ein energetischen Ladungsmuster nach der neuen Homöopathie nach Erich Körbler auf oder ist aus einem Material mit einem Lichtemissionsspektrum von 390 bis 740 nm ausgebildet.

## Patentansprüche

1. Kompresse (1) für die Applikation von Naturheilstoffen (5) und elektromagnetischen Strahlungen an einer Hautoberfläche (7) eines menschlichen oder tierischen Körpers, **dadurch gekennzeichnet, dass**
- die Kompresse aus Kautschuk, Silikon, Gummi oder einem vergleichbaren Kunststoff besteht;
- wahlweise flüssige, pastöse oder rieselförmige Naturheilstoffe (5) mit dem Kautschuk, Silikon, Gummi oder dem vergleichbaren Kunststoff vermischt, geschmolzen und zu einem einstückigen verformbaren Wirkstoffkörper mit Seitenflächen (3, 4) verformt sind,
- und mindestens eine Seitenfläche (3, 4) der Kompresse (1) wahlweise entweder mit einem energetischen Ladungsmuster nach der neuen Homöopathie nach E. Körbler versehen ist oder der Wirkstoffkörper aus einem Material besteht, der ein Lichtemissionsspektrum von 390 bis 740nm aufweist.

2. Kompresse (1') für die Applikation von Naturheilstoffen und elektromagnetischen Strahlungen an einer Hautoberfläche (7) eines menschlichen oder tierischen Körpers, **dadurch gekennzeichnet, dass**
- die Kompresse (1') aus Kautschuk, Silikon, Gummi oder eine vergleichbaren Kunststoff besteht,
- die Kompresse (1') aus einer Kammeranordnung (8) aus mehreren voneinander abgeschlossenen Kammern (9) und einer Wirkstoffabgabeschicht (10) an mindestens einer Seitenfläche (3, 4) der Kompresse (1') besteht, wobei wahlweise flüssige, pastöse oder rieselförmige Naturheilstoffe (5) in den abgeschlossenen Kammern (9) unverlierbar einbringbar sind und
- mindestens eine der Seitenflächen (3, 4) der Kompresse (1') wahlweise entweder mit einem energetischen Ladungsmuster nach der neuen Homöopathie nach E. Körbler versehen ist oder die Kompresse (1') aus einem Material besteht, der ein Lichtemissionsspektrum von 390 bis 740nm aufweist.

3. Kompresse nach Anspruch 1, **dadurch gekennzeichnet, dass** auf einer Seitenfläche (4) Öffnungen (6) vorgesehen sind.

4. Kompresse nach mindestens einem der vorgenannten Ansprüche, **dadurch gekennzeichnet, dass** es sich bei den Naturheilstoffen um Bachblüten handelt.

5. Kompresse nach mindestens einem der vorgenannten Ansprüche, **dadurch gekennzeichnet, dass** ein Verschlusselement einteilig mit der übrigen Kompresse ausgebildet ist.

6. Kompresse nach Anspruch 5, **dadurch gekennzeichnet, dass** sich die Teile des Verschlusselements seitlich von der Kompresse wegstrecken.

7. Kompresse nach mindestens einem der vorgenannten Ansprüche, **dadurch gekennzeichnet, dass** die Kompresse aus einem wärmeschrumpfenden Kunststoff besteht.

8. Kompresse nach mindestens einem der vorgenannten Ansprüche, **dadurch gekennzeichnet, dass** die Kompresse eine Form aufweist, die zumindest einen Teil der Konturen eines menschlichen oder tierischen Körperteils entspricht.

9. Kompresse nach mindestens einem der vorgenannten Ansprüche, **dadurch gekennzeichnet, dass** die Vorrichtung kissenförmig ausgebildet ist.

10. Kompresse nach mindestens einem der vorgenannten Ansprüche, **dadurch gekennzeichnet, dass** die Kompresse aus einem Kunststoff besteht, der recyclebar ist.

11. Kompresse nach mindestens einem der vorgenannten Ansprüche, **dadurch gekennzeichnet, dass** die Kompresse aus einem Kunststoff besteht, der UVbeständig ist.

12. Verfahren zur Herstellung einer Kompresse für die Applikation von Naturheilstoffen (5) und elektromagnetischen Strahlungen an der Hautoberfläche (7) eines menschlichen oder tierischen Körpers, **gekennzeichnet durch** folgende Verfahrensschritte:
a) Vermischen von Naturheilstoffen (5) und einem Kunststoffgranulat,
b) Aufschmelzen des Kunststoffgranulats,
c) Einfüllen des aufgeschmolzenen Kunststoffgranulats und der darin enthaltenen Naturheilstoffe in ein Form zur Bildung eines Wirkstoffkörpers und
d) mindestens eine der Seitenflächen (3, 4) der Kompresse (1') wahlweise entweder mit einem energetischen Ladungsmuster nach dem neuen Homöopathie nach E. Körbler versehen ist oder der Wirkstoffkörper aus einem Material besteht, der ein Lichtemissionsspektrum von 390 bis 740nm versehen wird.

13. Verfahren nach Anspruch 12, **dadurch gekennzeichnet, dass** das aufgeschmolzene Kunststoffgranulat mit den Naturheilstoffen in eine Form giessbar ist.

14. Verfahren nach Anspruch 12, **dadurch gekennzeichnet, dass** das aufgeschmolzene Kunststoffgranulat mit den Naturheilstoffen in eine Form spritzbar ist.

15. Verfahren nach Anspruch 12, **dadurch gekennzeichnet, dass** das aufgeschmolzene Kunststoffgranulat mit den Naturheilstoffen in eine Form pressbar ist.

## Claims

1. Compress (1) for applying natural curatives (5) and electromagnetic rays to a skin surface (7) of a human or animal body, **characterized in that**
- the compress is made of natural rubber, silicone, vulcanized rubber or a comparable plastic;
- alternately liquid, pasty or free-flowing natural curatives (5) are mixed with the natural rubber, silicone, vulcanized rubber or the comparable plastic, melted and shaped into a one-piece deformable active substance body with side surfaces (3, 4),
- and at least one side surface (3, 4) of the compress (1) is alternately either provided with an energetic charge pattern according to the new homeopathy of E. Körbler or the active substance body is made from a material that has a light emission spectrum of 390 to 740 nm.

2. Compress (1') for applying natural curatives and electromagnetic rays to a skin surface (7) of a human or animal body, **characterized in that**
- the compress (1') is made of natural rubber, silicone, vulcanized rubber or a comparable plastic;
- the compress (1') is composed of a chamber arrangement (8), made up of a plurality of chambers (9) closed off from one another, and of an active substance release layer (10) on at least one side surface (3, 4) of the compress (1'), in which case liquid, pasty or free-flowing natural curatives (5) can alternately be introduced into and confined within the closed-off chambers (9), and
- at least one of the side surfaces (3, 4) of the compress (1') is alternately either provided with an energetic charge pattern according to the new homeopathy of E. Körbler or the compress (1') is made from a material that has a light emission spectrum of 390 to 740 nm.

3. Compress according to Claim 1, **characterized in that** openings (6) are provided on one side surface (4).

4. Compress according to at least one of the preceding claims, **characterized in that** the natural curatives are Bach flowers.

5. Compress according to at least one of the preceding claims, **characterized in that** a closure element is designed in one piece with the rest of the compress.

6. Compress according to Claim 5, **characterized in that** the parts of the closure element extend laterally away from the compress.

7. Compress according to at least one of the preceding claims, **characterized in that** the compress is made of a heat-shrinkable plastic.

8. Compress according to at least one of the preceding claims, **characterized in that** the compress has a shape that corresponds to at least some of the contours of a human or animal body.

9. Compress according to at least one of the preceding claims, **characterized in that** the device is pad-shaped.

10. Compress according to at least one of the preceding claims, **characterized in that** the compress is made of a plastic that can be recycled.

11. Compress according to at least one of the preceding claims, **characterized in that** the compress is made of a plastic that is UV-resistant.

12. Method for production of a compress for applying natural curatives (5) and electromagnetic rays to the skin surface (7) of a human or animal body, **characterized by** the following method steps:
a) mixing together natural curatives (5) and a plastic granulate,
b) melting the plastic granulate,
c) pouring the molten plastic granulate and the natural curatives contained therein into a mould for forming an active substance body, and
d) alternately either providing at least one of the side surfaces (3, 4) of the compress (1') with an energetic charge pattern according to the new homeopathy of E. Körbler or making the active substance body from a material that has a light emission spectrum of 390 to 740 nm.

13. Method according to Claim 1 2, **characterized in that** the molten plastic granulate with the natural curatives can be cast into a mould.

14. Method according to Claim 12, **characterized in that** the molten plastic granulate with the natural curatives can be injected into a mould.

15. Method according to Claim 12, **characterized in that** the molten plastic granulate with the natural curatives can be pressed into a mould.

## Revendications

1. Compresse (1) pour l'application de substances thérapeutiques naturelles (5) et de rayonnements électromagnétiques sur une surface de peau (7) d'un corps humain ou animal, **caractérisée en ce que**
- la compresse est à base de caoutchouc, silicone, caoutchouc vulcanisé ou d'une matière plastique comparable ;
- des substances thérapeutiques naturelles (6) au choix liquides, pâteuses ou aptes à l'écoulement sont mélangées avec le caoutchouc, le silicone, le caoutchouc vulcanisé ou la matière plastique comparable, fondues et moulées en un corps à principes actifs monolithique pouvant être mis en forme, à faces latérales (3, 4),
- et, au choix, soit au moins une face latérale (3, 4) de la compresse (1) est munie d'un type de charge énergétique selon la nouvelle homéopathie selon E. Körbler, soit le corps à principes actifs consiste en un matériau qui présente un spectre d'émission de lumière de 390 à 740 nm.

2. Compresse (1') pour l'application de substances thérapeutiques naturelles (5) et de rayonnements électromagnétiques sur une surface de peau (7) d'un corps humain ou animal, **caractérisée en ce que**
- la compresse (1') est à base de caoutchouc, silicone, caoutchouc vulcanisé ou d'une matière plastique comparable ;
- la compresse (1') est constituée d'un système de chambres (8) composé de plusieurs chambres (9) séparées les unes des autres et d'une couche de libération de principes actifs (10) sur au moins une face latérale (3, 4) de la compresse (1'), de sorte que des substances thérapeutiques naturelles (5) au choix liquides, pâteuses ou aptes à l'écoulement peuvent être introduites sans perte dans les chambres (9) séparées et
- au moins l'une des faces latérales (3, 4) de la compresse (1') au choix soit est munie d'un type de charge énergétique selon la nouvelle homéopathie selon E. Körbler, soit la compresse (1') consiste en un matériau qui présente un spectre d'émission de lumière de 390 à 740 nm.

3. Compresse selon la revendication 1, **caractérisée en ce que** des ouvertures (6) sont prévues sur une face latérale (4).

4. Compresse selon au moins l'une des revendications précédentes, **caractérisée en ce que** les substances thérapeutiques naturelles consistent en élixirs floraux de Bach.

5. Compresse selon au moins l'une des revendications précédentes, **caractérisée en ce que** un élément de fermeture est constitué en partie par la compresse restante.

6. Compresse selon la revendication 5, **caractérisée en ce que** les parties de l'élément de fermeture s'éloignent latéralement de la compresse.

7. Compresse selon au moins l'une des revendications précédentes, **caractérisée en ce que** la compresse est constituée d'une matière plastique rétrécissant à la chaleur.

8. Compresse selon au moins l'une des revendications précédentes, **caractérisée en ce que** la compresse présente une forme qui correspond au moins à une partie des contours d'une partie du corps humain ou animal.

9. Compresse selon au moins l'une des revendications précédentes, **caractérisée en ce que** le dispositif est en forme de coussin.

10. Compresse selon au moins l'une des revendications précédentes, **caractérisée en ce que** la compresse est constituée d'une matière plastique qui est recyclable.

11. Compresse selon au moins l'une des revendications précédentes, **caractérisée en ce que** la compresse est constituée d'une matière plastique qui est résistante aux UV.

12. Procédé pour la fabrication d'une compresse pour l'application de substances thérapeutiques naturelles (5) et de rayonnements électromagnétiques sur la surface de peau (7) d'un corps humain ou animal, **caractérisé par** les étapes de processus suivantes :
a) mélange de substances thérapeutiques naturelles (5) et d'un produit granulé en matière plastique,
b) fusion du produit granulé en matière plastique,
c) introduction dans un moule du produit granulé en matière plastique fondu et des substances thérapeutiques naturelles contenues dans celui-ci, pour la formation d'un corps à principes actifs et
d) au moins l'une des faces latérales (3, 4) de la compresse (1') au choix soit est munie d'un type de charge énergétique selon la nouvelle homéopathie selon E. Körbler, soit le corps à principes actifs consiste en un matériau qui présente un spectre d'émission de lumière de 390 à 740 nm.

13. Procédé selon la revendication 1 2, **caractérisé en ce que** le produit granulé en matière plastique fondu, comportant les substances thérapeutiques naturelles, peut être versé dans un moule.

14. Procédé selon la revendication 12, **caractérisé en ce que** le produit granulé en matière plastique fondu, comportant les substances thérapeutiques naturelles, peut être pulvérisé dans un moule.

15. Procédé selon la revendication 12, **caractérisé en ce que** le produit granulé en matière plastique fondu, comportant les substances thérapeutiques naturelles, peut être pressé dans un moule.
